# FASCICULE DE BREVET EUROPEEN

(11) **EP 1 629 123 B1**
(45) Date de publication et mention de la délivrance du brevet: **12.11.2008**
(21) Numéro de dépôt: 04785651.3
(22) Date de dépôt: 02.06.2004
(51) Int. Cl.: C12Q 1/68, G01N 33/68

(54) **PROCEDE DE DIAGNOSTIC ET/OU DE PRONOSTIC D UN SYNDROME SEPTIQUE**
VERFAHREN ZUR DIAGNOSE UND/ODER PROGNOSE EINES SEPTISCHEN SYNDROMS
METHOD FOR DIAGNOSIS AND/OR PROGNOSIS OF A SEPTIC SYNDROME

(30) Priorité: 03.06.2003 FR 0306660
(43) Date de publication de la demande: 01.03.2006
(73) Titulaire: BIOMERIEUX, 69280 Marcy-L'Etoile (FR)
(72) Inventeur: PACHOT, Alexandre, F-69650 QUINCIEUX (FR)
(86) Numéro de dépôt international: PCT/FR2004/050210
(87) Numéro de publication internationale: WO 2004/108957

(56) Documents cités:
- EP-A- 1 310 567
- WO-A-20/04005539
- US-B1- 6 303 321
- STUHLMÜLLER B ET AL: "Identification of known and novel genes in activated monocytes fom patients with rheumatoid arthritis" ARTHRITIS AND RHEUMATISM, LIPPINCOTT, PHILADELPHIA, US, vol. 43, no. 4, avril 2000 (2000-04), pages 775-790, XP002255228 ISSN: 0004-3591
- CASEY L.C ET AL.: "Plasma cytokine and endotoxin levels correlate with survival in patients with the sepsis syndrome" ANNALS OF INTERNAL MEDICINE, vol. 119, no. 8, 15 octobre 1993 (1993-10-15), pages 771-778, XP008026477 cité dans la demande
- VAN DER POLL T. ET AL.: "Antiinflammatory cytokine responses during clinical sepsis and experimental endotoxemia: sequential measurements of plasma soluble interleukin (IL)-1 receptor type II, IL-10, and IL-13" THE JOURNAL OF INFECTIOUS DISEASES, vol. 175, 1997, pages 118-122, XP008026473 cité dans la demande
- THIJS L.G. AND HACK C.E.: "Time course of cytokine levels in sepsis" INTENSIVE CARE MEDICINE, vol. 21, 1995, pages S258-S263, XP008026472 cité dans la demande
- BERNER R. ET AL.: "Plasma levels and gene expression of granulocyte colony-stimulating factor, tumor necrosis factor-alpha, interleukin (IL)-1 beta, IL-6, IL-8, and soluble intercellular adhesion molecule-1 in neonatal early onset sepsis." PEDIATRIC RESEARCH, vol. 44, no. 4, octobre 1998 (1998-10), pages 469-477, XP008026542
- LEE K H: "Proteomics: a technology-driven and technology-limited discovery science" TRENDS IN BIOTECHNOLOGY, ELSEVIER, AMSTERDAM,, GB, vol. 19, no. 6, 1 juin 2001 (2001-06-01), pages 217-222, XP004239791 ISSN: 0167-7799

## Description

La présente invention concerne un procédé de diagnostic et/ou de pronostic d'un syndrome septique. Un kit de diagnostic et/ou de pronostic d'un syndrome septique est également décrit.
Le syndrome septique, réponse systémique à l'infection, représente l'une des premières causes de mortalité dans les services de réanimation. Il peut résulter d'une infection bactérienne, virale, mycosique ou parasitaire. Parmi ce syndrome septique, on distingue par ordre croissant de gravité le scpsis, le sepsis sévère et le choc septique. Un groupe d'experts a ainsi proposé en 1992 des critères de définitions de ces trois syndromes cliniques (R. C. Bone et al, The ACCP/SCCM Consensus Conference Committee. American College of Chest Physicians/Society of Critical Care Medicine. Chest 101 (6):1644-1655,1992) :
• le sepsis est ainsi une réponse systémique inflammatoire liée à une infection,
• un sepsis sévère est un sepsis accompagné du disfonctionnement d'au moins un organe,
• le choc septique est un sepsis sévère associé à une hypotension persistante et peut être qualifié par :
   o la présence d'un site infectieux identifié,
   o une réponse inflammatoire généralisée se manifestant par au moins deux des signes suivants : a) température supérieure à 38°C ou inférieure à 36°C ; b) rythme cardiaque supérieur à 90 battements par minute ; c) rythme respiratoire supérieur à 20 respirations par minute ; d) nombre de leucocytes supérieur à 12000/mm³ ou inférieur à 4000/mm³,
   o une hypotension persistante malgré des remplissages adéquats et des traitements vasopresseurs.

D'une manière générale, les signes d'un sepsis, d'un sepsis sévère et d'un choc septique sont proches, et la différence entre ces 3 situations réside principalement dans l'importance de la perturbation de toutes les fonctions vitales. Lors d'un choc septique, on observe principalement une chute de pression artérielle, tachycardie, polypnée, marbrures cutanées, hypo- ou hyperthermie, frissons. Ces signes s'accompagnent également d'un dysfonctionnement d'organes "cibles" avec une altération de fonction d'organes à distance du foyer infectieux (reins, poumons, système nerveux central, appareil digestif et système hématologique le plus souvent atteints) se traduisant par une oligurie (<0,5 ml/kg/h), insuffisance rénale, hypoxémie, thrombopénie, agitation , confusion.

L'évolution d'un syndrome septique depuis le stade de sepsis vers un stade de sepsis sévère, puis de choc septique n'est pas systématique puisque environ 64% des patients septiques développent un sepsis sévère, et 23% des patients en sepsis sévère évoluent en choc septique. Avant cette étape ultime de choc septique, des traitements doivent être prescrits au patient afin d'interrompre et d'inverser le processus physiopathologique. Il faut ainsi restaurer un état hémodynamique satisfaisant et assurer une ventilation efficace. Il faut également mener de front le traitement symptomatique du choc et un traitement antibiotique adapté dès que possible aux données bactériologiques.

Il apparaît ainsi que si certains patients développant un syndrome septique, et notamment un choc septique, peuvent être réanimés par une prise en charge standard, telle qu'un traitement antibiotique de large spectre mis en place avant les résultats des analyses bactériologiques indiquant la source infectieuse, d'autres patients, développant un syndrome septique beaucoup plus grave, nécessitent une mise en place de thérapeutiques lourdes, comme la protéine C activée. Au dela du coût très élevé, ce genre de thérapeutique expose les patients à des risques d'effets indésirables très importants (troubles de la coagulation ...). Il est donc très important de cibler de manière efficace les patients suceptibles de bénéficier d'un tel traitement.

De ce fait, le diagnostic précoce d'un syndrome septique est essentiel et permet de proposer un traitement adapté au patient. De plus, le pronostic du syndrome septique, et notamment d'un choc septique est primordial pour proposer à chaque patient un traitement adapté, et discriminer au plus tôt les patients présentant un syndrome septique de mauvais pronostic, et qui nécessitent une thérapie lourde, des patients de bon pronostic.

Actuellement, le diagnostic et le pronostic d'un syndrome septique, et notamment d'un choc septique sont essentiellement basés sur le nombre des défaillances viscérales, la réponse au traitement symptomatique, l'accessibilité à la thérapeutique médicale et/ou chirurgicale du foyer infectieux initial et des éventuels foyers métastatiques.

Ceci présente toutefois l'inconvénient de n'être applicable qu'à un stade avancé du syndrome septique, et notamment du choc septique, réduisant les chances de survie du patient.

Le diagnostic et le pronostic d'un syndrome septique peuvent également être basés sur la détection de certaines protéines ou facteurs solubles impliquées dans ce syndrome, telles que les cytokines. On peut notamment citer la publication de Thijs & Hack (Intensive Care Med, 1995, 21 : S258-S263), qui présente une revue des concentrations plasmatiques de certaines cytokines telles que TNF, IL-1b, IL-6, IL-8, lors d'un sepsis. Ainsi, le dosage de cytokines, impliquées lors du développement d'un syndrome septique peut être un moyen de diagnostic et de pronostic d'un syndrome septique. Ces auteurs ont ainsi décrit une corrélation positive entre la teneur plasmatique d'IL-1 (Interleukine-1) et un syndrome septique de mauvais pronostic (Thijs & Hack, Intensive Care Med 31 :S258-263, 1995). Toutefois, d'autres auteurs n'ont trouvé aucune corrélation entre l'IL-1 et un mauvais pronostic du syndrome septique, suggérant une grande variabilité de ce facteur. De plus, des dosages élevés de TNF (Tumor Necrosis Factor) ont été associés aussi à un mauvais pronostic (Casey et al., Ann Intern Med. 1993. 119:771-778). Le TNF-α puis l'IL-1β sont les deux premières cytokines pro-inflammatoire libérées par les monocytes après le déclenchement d'un état septique.

D'autres auteurs ont montré que la teneur en IL-10 (Interleukine-10) plasmatique est plus élevée chez les patients développant un sepsis de mauvais pronostic alors qu'elle décroît significativement chez les patients présentant un sepsis de bon pronostic pour ne pas être détectable chez les patients sains (Van der Poll, J. Infect. Dis. 175 :118-122, 1997). L'IL-10 est une cytokine anti inflammatoire très importante, et qui, par sa capacité à inhiber la production du TNF-α et de l'IL-1β, participe à la mise en place de l'état d'immuno-paralysie. Toutefois, cette augmentation de la teneur en IL-10 n'étant détectable que chez 80% des patients en choc septique, la détection unique de ce facteur reste insuffisante pour pronostiquer l'évolution du choc septique.

On peut citer également le brevet US-B-6,303,321 qui décrit une méthode de pronostic de la sévérité d'un syndrome septique comprenant la mesure de la concentration sérique en HMG1 (high mobility group 1 protein) par une technique immunoblot. L'HMG1 est, à l'inverse du TNF-α et de l'IL-1β, décrit comme un médiateur pro-inflammatoire tardif des syndromes septiques. Une forte concentration en HMG1 est corrélée à un mauvais pronostic, la concentration sérique en HMG1 n'étant pas détectée chez les patients sains. Il a par contre été décrit chez la souris une régulation post transcriptionnelle du gène HMG1, suggérant que l'expression de ce gène ne doit être analysé qu'au niveau protéique (Wang et al, Science, 1999, vol 285, p248-251).

EP-A-1 310 567 (OLIGENE GMBH, 2003-05-14) décrit un procédé de diagnostic et/ou de pronostic d'un syndrome septique ([0015], [0046]) selon lequel on dispose d'un échantillon biologique du patient (échantillon sanguin; "Patientenmaterial (Blut oder Gewebe)", [0046]) et on extrait les acides nucléiques de l'échantillon ("cRNA [...] oder cDNA Sonden, die dem Patientenmaterial [...] entstammen", [0046]), on dispose d'au moins quatre réactifs spécifiques comprenant des réactifs spécifiques des gènes cibles IL-1beta, IL-10, TGFbeta (sondes d'hybridation ([0046]); Tableau 1), on détermine l'expression d'au moins quatre gènes cibles dont IL-1beta, IL-10, TGFbeta(Tableau 1; [0046]).

Il est toutefois important de noter que, actuellement, aucune cytokine n'est reconnue de manière consensuelle comme outil de diagnostic ou de pronostic d'un syndrome septique. De plus, le dosage plasmatique de cytokines est basée sur une réaction antigène anticorps qui peut être faussée par la présence de réaction croisée avec d'autres antigènes non spécifiques des cytokines ou par la présence de cytokines complexé dans le plasma a un récepteur soluble.
La présente invention se propose de résoudre les inconvénients de l'état de la technique en présentant un nouvel outil fiable de diagnostic et/ou de pronostic d'un syndrome septique, tel que notamment un choc septique.
D'une manière surprenante, les inventeurs ont mis en évidence que le diagnostic et/ou le pronostic d'un syndrome septique peut être déterminé par l'étude d'un panel de gènes sélectionnés parmi IL-10, TGFβ, HMG1, T-bet (T cell specific T-box transcritption factor
T bet), IL-1β, GATA-3 (GATA - binding protein 3), TNF-α. De plus, l'analyse en une seule étape de l'expression de plusieurs de ces gènes permet d'obtenir un outil très efficace pour le pronostic et/ou diagnostic d'un syndrome septique.

Avant d'aller plus avant, les définitions suivantes sont données afin de mieux comprendre l'invention.
On entend par syndrome septique, une réponse systémique à l'infection. Ce syndrome septique peut être à un stade de sepsis, sepsis sévère ou de choc septique. Préférentiellement, le syndrome septique est un choc septique.
On entend par échantillon biologique, tout matériel issu du patient, susceptible de contenir un matériel biologique permettant de détecter l'expression d'un gène, et peut être notamment un échantillon de sang, de sérum, de salive ou de tissu ou de cellules circulantes du patient. On dispose de cet échantillon biologique par tout type de prélèvement connu de l'homme du métier, tel que notamment une prise de sang.
On entend par matériel biologique tout matériel permettant de détecter l'expression d'un gène, comme notamment un acide nucléique ou une protéine. L'acide nucléique peut être notamment un ARN (acide ribonucléique) tel qu'un ARNm (ARN messager). Selon un mode préféré de réalisation de l'invention, le matériel biologique est un acide nucléique, et encore plus préférentiellement un ARNm (ARN messager).
L'extraction du matériel biologique de l'échantillon biologique lors de l'étape a) peut être réalisée par tous les protocoles d'extraction d'acides nucléiques ou de protéines bien connus de l'homme du métier.
A titre indicatif, l'extraction d'acides nucléique peut notamment être réalisée par :
• une étape de lyse des cellules présentes dans l'échantillon biologique, afin de libérer les acides nucléiques contenus dans les enveloppes protéiques et/ou lipidiques des microorganismes (comme des débris cellulaires qui perturbent les réactions ultérieures). A titre d'exemple, on peut utiliser les méthodes de lyse telles que décrite dans les demandes de brevet de la Demanderesse :
   o WO-A-00/05338 sur la lyse mixte magnétique et mécanique,
   o WO-A-99/53304 sur la lyse électrique, et
   o WO-A-99/15321 sur la lyse mécanique.
   L'homme du métier pourra utiliser d'autres méthodes de lyse bien connues, telles que les chocs thermiques ou osmotiques ou les lyses chimiques par des agents chaotropiques tels que les sels de guanidium (US-A-5,234,809).
• une étape de purification, permettant la séparation entre les acides nucléiques et les autres constituants cellulaires relargués dans l'étape de lyse. Cette étape permet généralement de concentrer les acides nucléiques. A titre d'exemple, on peut utiliser des particules magnétiques éventuellement revêtues d'oligonucléotides, par adsorption ou covalence (voir à ce sujet les brevets US-A-4,672,040 et US-A-5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet déposées par la Demanderesse sous les références suivantes : WO-A-97/45202 et
WO-A-99/35500 . Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep^{®} Silica, Promega: MagneSil^{™} Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind^{™}).
On entend par réactif spécifique un réactif qui réagit avec le matériel biologique afin de mettre en évidence, d'une manière directe ou indirecte l'expression d'un gène cible, qui peut être déterminé par l'analyse des ARNm issus de ce gène, ou par l'analyse de la protéine codée par ce gène.

A titre indicatif, lorsque l'on souhaite déterminer l'expression d'un gène cible par l'analyse de la proteine codée par ce gène, ce réactif spécifique comprend au moins un anticorps spécifique de la protéine codée par ce gène cible.
A titre indicatif, lorsque l'on souhaite déterminer l'expression d'un gène cible par l'analyse des ARNm transcrits à partir de ce gène, ce réactif spécifique comprend au moins une amorce d'amplification spécifique de l'ADN complémentaire de cet ARNm (on parle alors d'amorce d'amplification spécifique d'un gène cible). L'ADN complémentaire d'un ARNm peut être obtenu selon un protocole bien connu de l'homme du métier. A titre indicatif, on extrait lors de l'étape a) du procédé selon l'invention les ARN totaux (comprenant les ARN ribosomaux, les ARN de transfert, les ARNm) de l'échantillon biologique. On réalise ensuite une réaction de transcription reverse à l'aide d'une enzyme reverse transcriptase qui permet d'obtenir, à partir d'un fragment d'ARN, un fragment complémentaire d'ADN. La réalisation d'une telle étape est bien connue de l'homme du métier. Lorsque l'on souhaite plus particulierement obtenir uniquement les ADN complémentaires des ARN messagers, on réalise cette étape enzymatique en présence de fragments nucléotidiques comprenant uniquement des bases thymine (polyT), qui s'hybrident par complémentarité sur la séquence polyA des différents ARNm afin de former un complexe polyT-polyA qui sert alors de point de départ à la réaction de transcription reverse réalisée par l'enzyme reverse transcriptase. On obtient alors différents ADN complémentaires des différents ARN messagers initialement présents dans échantillon biologique. Dans la suite de l'exposé, on entend par ADNc, un ADN complementaire d'un ARN messager.
Par amorce d'amplification, on entend un fragment nucléotidique comprenant de 5 à 100 motifs nucléotidiques, préférentiellement de 15 à 25 motifs nucléotidiques et possédant une spécificité d'hybridation dans des conditions déterminées pour l'initiation d'une polymérisation enzymatique, par exemple dans une réaction d'amplification enzymatique.
Par réaction d'amplification enzymatique, on entend un processus générant de multiples copies d'un fragment nucléotidique cible à l'aide d'amorces d'amplification spécifiques par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US-A-4,683,195, US-A-4,683,202 et US-A-4,800,159,
- LCR (Ligase Chain Reaction), exposée par exemple dans la demande de brevet EP-A-0 201 184,
- RCR (Repair Chain Reaction), décrite dans la demande de brevet WO-A-90/01069,
- 3SR (Self Sustained Sequence Replication) avec la demande de brevet WO-A-90/06995,
- NASBA (Nucleic Acid Sequence-Based Amplification) avec la demande de brevet WO-A-91/02818, et
- TMA (Transcription Mediated Amplification) avec le brevet US-A-5,399,491.
On parle alors d'amplicons pour désigner les polynucléotides générés par une technique d'amplification enzymatique. Préférentiellement, lorsque l'amplification enzymatique est une PCR, le réactif spécifique comprend au moins 2 amorces d'amplification spécifiques afin d'amplifier une région particulière de l'ADN complémentaire de l'ARNm issu du gène cible. Lorsque l'amplification enzymatique est une PCR réalisée après une réaction de transcription reverse, on parle de RT-PCR.
Par sonde d'hybridation, on entend un fragment nucléotidique comprenant de 5 à 100 motifs nucléotidiques, notamment de 6 à 35 motifs nucléotidiques, possédant une spécificité d'hybridation dans des conditions déterminées pour former un complexe d'hybridation avec un fragment nucléotidique cible. Dans la présente invention, le fragment nucléotidique cible peut être une séquence nucléotidique comprise dans un ARN messager ou une séquence nucléotidique comprise dans un ADN complémentaire obtenu par transcription inverse dudit ARN messager.
Par hybridation, on entend le processus au cours duquel, dans des conditions appropriées, deux fragments nucléotidiques, tels que par exemple une sonde d'hybridation et un fragment nucléotidique cible, ayant des séquences suffisamment complémentaires sont susceptibles de former un double brin avec des liaisons hydrogènes stables et spécifiques.
Un fragment nucléotidique "capable de s'hybrider" avec un polynucléotide est un fragment pouvant s'hybrider avec ledit polynucléolide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.
Par détection on entend soit une détection directe par une méthode physique, soit une méthode de détection à l'aide d'un marqueur. De nombreuses méthodes de détection existent pour la détection des acides nucléiques. [Voir par exemple Kricka et al., Clinical Chemistry, 1999, n° 45(4), p.453-458 ou Keller G.H. et al., DNA Probes, 2nd Ed., Stockton Press, 1993, sections 5 et 6, p.173-249]. Par marqueur, on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la bêtagalactosidase, la glucose-6-phosphate déshydrogénase ; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I. Ainsi, le polynucléotide peut être marqué pendant l'étape d'amplification enzymatique, par exemple en utilisant un nucléotide triphosphate marqué pour la réaction d'amplification. Le nucléotide marqué sera un désoxyribonucléotide dans les systèmes d'amplification générant un ADN, comme la PCR, ou un ribonucléotide dans les techniques d'amplification générant un ARN, comme les techniques TMA ou NASBA. Le polynucléotide peut aussi être marqué après l'étape d'amplification, par exemple en hybridant une sonde marquée selon la technique d'hybridation sandwich décrite dans le document WO-A-91/19812.
Un autre mode particulier préférentiel de marquage d'acides nucléiques est décrit dans la demande FR-A-2 780 059 de la Demanderesse. Un autre mode préférentiel de détection utilise l'activité exonucléase 5'-3' d'une polymérase, tel que décrit par Holland P.M., PNAS (1991) p 7276-7280.
Des systèmes d'amplification du signal peuvent être utilisés comme décrit dans le document WO-A-95/08000 et, dans ce cas, la réaction préliminaire d'amplification enzymatique peut ne pas être nécessaire.
Au sens de la présente invention, la sonde d'hybridation peut être une sonde dite de capture. Dans ce cas, le fragment nucléotidique cible peut être préalablement marqué au moyen d'un marqueur. La sonde dite de capture est immobilisée ou immobilisable sur un support solide par tout moyen approprié, c'est-à-dire directement ou indirectement, par exemple par covalence ou adsorption. On réalise alors une réaction d'hybridation entre ladite sonde de détection et le fragment nucléotidique cible marqué.
La sonde d'hybridation peut également être une sonde dite de détection. Dans ce cas, la sonde d'hybridation peut être marquée au moyen d'un marqueur. On réalise alors une réaction d'hybridation entre ladite sonde de capture et le fragment nucléotidique cible.
Que l'on utilise une sonde dite de capture ou une sonde dite de détection, la réaction d'hybridation peut être réalisée sur un support solide qui inclut tous les matériaux sur lesquels peut être immobilisé un acide nucléique. Des matériaux de synthèse ou des matériaux naturels, éventuellement modifiés chimiquement, peuvent être utilisés comme support solide, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule ou d'une biopuce. Par biopuce, on entend un support solide de dimension réduite où sont fixée une multitude de sondes de capture à des positions prédéterminées. Le concept de biopuce, plus précisément de puce à ADN, date du début des années 90. De nos jours, ce concept s'est élargi puisque des puces à protéines commencent à se développer. Il repose sur une technologie pluridisciplinaire intégrant la micro-électronique, la chimie des acides nucléiques, l'analyse d'images et l'informatique. Le principe de fonctionnement repose sur un fondement de la biologie moléculaire : le phénomène d'hybridation, c'est-à-dire l'appariement par complémentarité des bases de deux séquences d'ADN et/ou d'ARN. La méthode des biopuces repose sur l'emploi de sondes de capture fixées sur un support solide sur lesquelles on fait agir un échantillon de fragments nucléotidiques cibles marqués directement ou indirectement avec des fluorochromes. Les sondes de capture sont positionnées de manière spécifique sur le support ou puce et chaque hybridation donne une information particulière, en relation avec le fragment nucléotidique cible. Les informations obtenues sont cumulatives, et permettent par exemple de quantifier le niveau d'expression d'un gène ou de plusieurs gènes cibles. Pour analyser l'expression d'un gène cible, on peut alors réaliser une biopuce portant de très nombreuses sondes qui correspondent à tout ou partie du gène cible, qui est transcrit en ARNm. On hybride alors par exemple les ADN complémentaires des ARNm issues du ou des gènes cibles que l'on souhaite analyser. Après hybridation, le support ou puce est lavé(e), lu(e) par exemple par un scanner et l'analyse de la fluorescence est traitée par informatique. On peut citer à titre indicatif, les puces à ADN mises au point par la société Affymetrix ("Accessing Genetic Information with High-Density DNA arrays", M. Chee et al., Science, 1996, 274, 610-614. "Light-generated oligonucleotide arrays for rapide DNA sequence analysis", A. Caviani Pease et al., Proc. Natl. Acad. Sci. USA, 1994, 91, 5022-5026), pour les diagnostics moléculaires. Dans cette technologie, les sondes de capture sont généralement de tailles réduites, autour de vingt nucléotides. D'autres exemples de biopuces sont donnés dans les publications de G. Ramsay, Nature Biotechnology, 1998, n°16, p. 40-44 ; F. Ginot, Human Mutation, 1997, n°10, p.1-10 ; J. Cheng et al, Molecular diagnosis, 1996, n°1(3), p.183-200 ; T. Livache et al, Nucleic Acids Research, 1994, n° 22(15), p. 2915-2921 ; J. Cheng et al, Nature Biotechnology, 1998, n° 16, p. 541-546 ou dans les brevets US-A-4,981,783, US-A-5,700,637, US-A-5,445,934, US-A-5,744,305 et US-A-5,807,522. La caractéristique principale du support solide doit être de conserver les caractéristiques d'hybridation des sondes de capture sur les fragments nucléotidiques cibles tout en générant un bruit de fond minimum pour la méthode de détection.
Lors de la présente invention, la détermination de l'expression de gènes cibles peut être analysée par l'expression des ARNm qui sont transcrits à un moment donné. Dans ce cas, le matériel biologique est un acide nucléique, et le réactif spécifique peut être indifféremment une amorce d'amplification ou une sonde d'hybridation telles que définies précédemment. L'expression d'un gène cible peut être également analysé par l'expression des protéines codées par le gène cible. Dans ce cas, le matériel biologique est une protéine et le réactif spécifique peut être un anticorps spécifique de la protéine codée par le gène cible.
A titre indicatif, on peut déterminer l'expression d'un gène cible de la manière suivante:
1) après avoir extrait les ARN totaux d'un échantillon biologique tel que défini lors de l'étape a) du procédé selon l'invention telle que présentée précédemment, on réalise une étape de transcription reverse, telle que décrite précedemment afin d'obtenir les différents ADN complémentaires des différents ARN messagers initialement présents dans l'échantillon biologique (ou ADNc)
2) on amplifie spécifiquement les ADNc. Dans ce cas, le réactif spécifique utilisé comprend au moins une amorce d'amplication spécifique du gène cible, telle que définie précédemment. Cette étape peut être réalisée notamment par une réaction d'amplification de type PCR ou par toute autre technique d'amplification telle que définie précédemment. On peut également amplifier simultanément les ADNc de plusieurs gènes cibles : on parle alors d'amplification en multiplex.
3) on détermine l'expression du gène cible en quantifiant les ADNc. Les ADNc peuvent être quantifiés notamment par l'utilisation d'une gamme de quantification obtenue par une réaction d'amplification conduite jusqu'à saturation. Afin de tenir compte de la variabilité d'efficité enzymatique qui peut être observée lors des différentes étapes (transcription reverse, PCR...), on peut normaliser l'expression du gène cible des différents groupes de patients, par la détermination simultanée de l'expression d'un gène dit de ménage, dont l'expression est similaire chez les différents groupes de patients. En réalisant un rapport entre l'expression du gène cible et l'expression du gène de ménage, on corrige ainsi toute variabilité entre les différentes expérimentations. L'homme du métier pourra se referer notammant aux publications suivantes : Bustin SA Journal of molecular endocrinology, 2002, 29 : 23-39 ; Giulietti A Methods, 2001, 25 : 386-401.
On peut également déterminer l'expression d'un gène cible de la manière suivante:
1) après avoir extrait les ARN totaux d'un échantillon biologique tel que défini lors de l'étape a) du procédé selon l'invention telle que présentée précedemment, on réalise une étape de transcription reverse, telle que décrite précédemment afin d'obtenir les différents ADN complémentaires des différents ARN messagers initialement présents dans l'échantillon biologique (ou ADNc)
2) on immobilise les ADNc sur une membrane
3) on détermine l'expression du gène cible en hybridant les ADNc à des sondes d'hybridation spécifiques du gène cibl,e préalablement marquées. De telles techniques d'hybridation sont bien connues de l'homme du métier, et on peut citer notamment la technique du Northern blot. Cette réaction d'hybridation peut être réalisée après une étape d'amplification spécifique des ADN complémentaires des ARN messagers d'un gène cible lorsque notamment le gène est faiblement exprimé.
L'analyse de l'expression d'un gène cible permet alors de disposer d'un outil pour le diagnostic et / ou le pronostic d'un syndrome septique. On peut par exemple analyser l'expression d'un gène cible chez un patient dont on ne connaît pas le pronostic de son syndrome septique, et en comparant avec des valeurs d'expression moyenne connues du gène cible de patients de bon pronostic et des valeurs d'expression moyenne connues du gène cible de patients de mauvais pronostic, déterminer si le patient est de bon ou de mauvais pronostic afin de lui proposer un traitemen adapté. On peut également étudier simultanement l'expression de plusieurs gènes cibles, par notamment l'utilisation d'une biopuce et on détermine, en une seule étape, c'est à dire à partir d'un même prélèvement et par une seule analyse (à l'aide de dosage répétés ou de mesure en une seule étape), l'expression de plusieurs gènes cibles. Les gènes sont ensuite analysés de manière globale pour un patient dont on ne connaît pas le pronostic de son syndrome septique, et en comparant avec des valeurs d'expression moyenne connues d'un même panel de gènes cibles de patients de bon pronostic et des valeurs d'expression moyenne connues d'un même panel de gènes cibles de patients de mauvais pronostic, on peut donc déterminer si le patient est de bon ou de mauvais pronostic afin de lui proposer un traitemen adapté.

Ainsi, un procédé de diagnostic et/ou de pronostic d'un syndrome septique est décrit, selon lequel :
a. on dispose d'un échantillon biologique du patient et on extrait du matériel biologique de l'échantillon biologique
b. on dispose d'au moins quatre réactifs spécifiques sélectionnés parmi les réactifs spécifiques suivants: réactif spécifique du gène cible IL-10, réactif spécifique du gène cible TGFβ, réactif spécifique du gène cible HMG1, réactif spécifique du gène cible T-bet, réactif spécifique du gène cible IL-1β, réactif spécifique du gène cible TNFα, réactif spécifique du gène cible GATA-3
c. on détermine l'expression d'au moins quatre gènes cibles choisis parmi : IL-10, TGFβ, HMG1, T-bet, IL-1β, TNFα, GATA-3.
Selon un mode préféré de réalisation, l'échantillon biologique est un échantillon sanguin. Selon un mode préféré de réalisation, le matériel biologique est un acide nucléique.
Selon un mode préféré de réalisation, on extrait les ARN totaux de l'échantillon biologique.
Selon un mode préféré de réalisation, le réactif spécifiques de l'IL10 comprend au moins une amorce d'amplification spécifique du gène cible IL-10, le réactif spécifique de TGFβ comprend au moins une amorce d'amplification spécifique du gène cible TGFβ, le réactif spécifique de HMG1 comprend au moins une amorce d'amplification spécifique du gène cible HMG1, le réactif spécifique de T-bet comprend au moins une amorce d'amplification spécifique du gène cible T-bet, le réactif spécifique de l'IL-1β comprend au moins une amorce d'amplification spécifique du gène cible IL-1β, le réactif spécifique de TNFα comprend au moins une amorce d'amplification spécifique du gène cible TNFα et/ou le réactif spécifique de GATA 3 comprend au moins une amorce d'amplification spécifique du gène cible GATA 3.
Selon un autre mode de réalisation, le réactif spécifique comprend au moins une sonde d'hybridation spécifique d'un gène cible. Préférentiellement, le réactif spécifique de l'IL10 comprend au moins une sonde d'hybridation spécifique du gène cible IL-10, le réactif spécifique de TGFβ comprend au moins une sonde. d'hybridation spécifique du gène cible TGFβ, le réactif spécifique de HMG1 comprend au moins une sonde d'hybridation spécifique du gène cible HMG1, le réactif spécifique de T-bet comprend au moins une sonde d'hybridation spécifique du gène cible T-bet, le réactif spécifique de l'IL-1β comprend au moins une sonde d'hybridation spécifique du gène cible IL-1β, le réactif spécifique de TNFα comprend au moins une sonde d'hybridation spécifique du gène cible TNFα et/ou le réactif spécifique de GATA 3 comprend au moins une sonde d'hybridation spécifique du gène cible GATA 3. Cette sonde peut être une sonde de détection ou une sonde de capture, telle que notamment une sonde de capture immobilisée sur une biopuce.
L'invention concerne préférentiellement la détermination de l'expression des gènes cibles par l'analyse de l'expression des ARNm.
Selon un mode préféré de réalisation, on dispose lors de l'étape b) du procédé, d'au moins deux réactifs spécifiques sélectionnés parmi les réactifs spécifiques suivants : réactif spécifique du gène TGFβ, réactif spécifique du gène HMG1, et on détermine, lors de l'étape c) du procédé selon l'invention, l'expression d'au moins deux gènes cibles choisis parmi : TGFβ, HMG1.
Selon un mode préféré de réalisation, on dispose, lors de l'étape b) du procédé, d'au moins trois, préférentiellement d'au moins quatre et encore plus préférentiellement d'au moins cinq réactifs spécifiques sélectionnés parmi les réactifs spécifiques suivants : réactif spécifique du gène d'IL-10, réactif spécifique du gène TGFβ, réactif spécifique du gène HMG1, réactif spécifique du gène T-bet ; réactif spécifique du gène IL-1β, réactif spécifique du gène TNFα, réactif spécifique du gène GATA-3, et on détermine, lors de l'étape c) du procédé selon l'invention, l'expression d'au moins 3 préférentiellement d'au moins 4 et encore plus préférentiellement d'au moins 5 gènes choisis parmi IL-10, TGFβ, HMG1, T-bet, IL-1β, TNFα, GATA-3.
Selon un mode préféré de réalisation, on dispose lors de l'étape b) du procédé, d'au moins trois réactifs spécifiques sélectionnés parmi les réactifs spécifiques suivantes : réactif spécifique du gène TGFβ, réactif spécifique du gène HMG1, réactif spécifique du gène IL-1β et on détermine, lors de l'étape c) du procédé selon l'invention, l'expression d'au moins trois gènes cibles choisis parmi : TGFβ, HMG1, IL-1β.
Selon un autre mode préféré de réalisation, on dispose lors de l'étape b) du procédé, d'au moins quatre réactifs spécifiques sélectionnés parmi les réactifs spécifiques suivants : réactif spécifique du gène TGFβ, réactif spécifique du gène HMG1, réactif spécifique du gène IL-1β, réactif spécifique du gène d'IL-10 et on détermine, lors de l'étape c) du procédé selon l'invention, l'expression d'au moins quatre gènes cibles choisis parmi : TGFβ, HMG1, IL-1β, IL-10.
Selon un autre mode préféré de réalisation, on dispose lors de l'étape b) du procédé, d'au moins cinq réactifs spécifiques sélectionnés parmi les réactifs spécifiques suivants : réactif spécifique du gène TGFβ, réactif spécifique du gène HMG1, réactif spécifique du gène IL-1β, réactif spécifique du gène d'IL-10, réactif spécifique du gène T-bet et on détermine, lors de l'étape c) du procédé selon l'invention, l'expression d'au moins cinq gènes cibles choisis parmi : TGFβ, HMG1, IL-1β, IL-10, T-bet.
Un procédé de diagnostic et/ou de pronostic d'un syndrome septique, tel que défini précédemment, est également décrit, selon lequel :
a. on dispose d'un échantillon biologique du patient, tel que défini précédemment, et on extrait du matériel biologique, tel que défini précédemment, de l'échantillon biologique
b. on dispose d'au moins un réactif spécifique d'un gène choisi parmi les gènes suivants: IL-10, T-bet ; GATA-3.
c. on détermine l'expression, telle que définie précédemment, d'au moins un gène cible choisi parmi : IL-10 ; T-bet, GATA-3.
Selon un mode préféré de réalisation, l'échantillon biologique est un échantillon sanguin.

Selon un mode préféré de réalisation, le matériel biologique est un acide nucléique.
Selon un mode préféré de réalisation, le réactif spécifique est une amorce d'amplification, telle que définie précédemment, spécifique d'un gène choisi parmis IL-10 ; T-bet GATA 3.
Selon un autre mode préféré de réalisation, le réactif spécifique de IL-10 ; T-bet, ou de GATA 3 est une sonde d'hybridation telle que définie précédemment, spécifique respectivement des gènes cibles IL-10 ; T-bet, et GATA 3.
Un procédé de diagnostic et/ou de pronostic d'un syndrome septique est également concerné, selon lequel :
a. on dispose d'un échantillon biologique du patient et on extrait les ARN totaux de l'échantillon biologique
b. on dispose d'au moins un réactif spécifique du gène HMG1
c. on détermine l'expression des ARNm du gène HMG1.
Il est bien évident que dans ce procédé, on ne souhaite pas détecter l'expression du gène HMG1 au niveau proteique mais uniquement au niveau des ARNm. Préférentiellement, le réactif spécifique du gène HMG1 est une amorce d'amplification ou une sonde d'hybridation telle que définie précédemment.

Un kit de diagnostic et/ou de pronostic d'un syndrome septique est également concerné, comprenant au moins quatre réactifs spécifiques sélectionnés parmi les réactifs spécifiques suivants : réactif spécifique du gène d'IL-10, réactif spécifique du gène TGFβ, réactif spécifique du gène HMG1, réactif spécifique du gène T-bet ; réactif spécifique du gène IL-1β, réactif spécifique du gène TNFα, réactif spécifique du gène GATA-3.
Selon un mode préféré de réalisation, le réactif spécifique de l'IL10 comprend au moins une amorce d'amplification spécifique du gène cible IL-10, le réactif spécifique de TGFβ comprend au moins une amorce d'amplification spécifique du gène cible TGFβ, le réactif spécifique de HMG1 comprend au moins une amorce d'amplification spécifique du gène cible HMG1, le réactif spécifique de T-bet comprend au moins une amorces d'amplification spécifique du gène cible T-bet, le réactif spécifique de l'IL-1β comprend au moins une amorce d'amplification spécifique du gène cible IL-1β, le réactif spécifique de TNFα comprend au moins une amorce d'amplification spécifique du gène cible TNFα et/ou le réactif spécifique de GATA 3 comprend au moins une amorce d'amplification spécifique du gène cible GATA 3.
Selon un autre mode de réalisation, le réactif spécifique de l'IL10 comprend au moins une sonde d'hybridation spécifique du gène cible IL-10, le réactif spécifique de TGFβ comprend au moins une sonde d'hybridation spécifique du gène cible TGFβ, le réactif spécifique de HMG1 comprend au moins une sonde d'hybridation spécifique du gène cible HMG1, le réactif spécifique de T-bet comprend au moins une sonde d'hybridation spécifique du gène cible T-bet le réactif spécifique de l'IL-1β comprend au moins une sonde d'hybridation spécifique du gène cible IL-1β, le réactif spécifique de TNFα comprend au moins une sonde d'hybridation spécifique du gène cible TNFα et/ou le réactif spécifique de GATA 3 comprend au moins une sonde d'hybridation spécifique du gène cible GATA 3.
Est concerné également un kit de diagnostic et/ou de pronostic d'un syndrome septique comprenant au moins un réactif spécifique sélectionné parmi les réactifs spécifiques suivants : réactif spécifique du gène d'IL-10, réactif spécifique du gène T-bet ; réactif spécifique du gène GATA-3.
Selon un mode préféré de réalisation, le réactif spécifique de l'IL10 comprend au moins une amorce d'amplification spécifique du gène cible IL-10, le réactif spécifique de T-bet comprend au moins une amorce d'amplification spécifique du gène cible T-bet, et/ou le réactif spécifique de GATA 3 comprend au moins une amorce d'amplification spécifique du gène cible GATA 3.
Selon un autre mode préféré de réalisation, le réactif spécifique de l'IL10 comprend au moins une sonde d'hybridation spécifique du gène cible IL-10, le réactif spécifique de T-bet comprend au moins une sonde d'hybridation spécifique du gène cible T-bet, et/ou le réactif spécifique de GATA 3 comprend au moins une sonde d'hybridation spécifique du gène cible GATA 3.

Est concerné également un procédé de diagnostic et/ou de pronostic d'un syndrome septique selon lequel:
a. on dispose d'un échantillon biologique du patient et on extrait du matériel biologique de l'échantillon biologique
b. on dispose d'au moins deux réactifs spécifiques d'au moins deux gènes cibles choisi parmis : IL-10, TGFβ, HMG1, T-bet, IL-1β, TNFα, GATA-3.
c. on détermine, en une seule étape, l'expression d'au moins deux gènes cibles choisis parmi : IL-10, TGFβ, HMG1, T-bet, IL-1β, TNFα, GATA-3.
Selon un mode préféré de réalisation, l'échantillon biologique est un échantillon sanguin.
Selon un mode préféré de réalisation, on extrait les ARN totaux de l'échantillon biologique.
Le gène cible est choisi parmi IL-10, TGFβ, HMG1, T-bet, IL-1β, TNFα, GATA-3.
Selon un mode préféré de réalisation, le réactif spécifique de l'IL10 comprend au moins une amorce d'amplification spécifique du gène cible IL-10, le réactif spécifique de TGFβ comprend au moins une amorce d'amplification spécifique du gène cible TGFβ, le réactif spécifique de HMG1 comprend au moins une amorce d'amplification spécifique du gène cible HMG1, le réactif spécifique de T-bet comprend au moins une amorce d'amplification spécifique du gène cible T-bet, le réactif spécifique de l'IL-1β comprend au moins une amorce d'amplification spécifique du gène cible IL-1β, le réactif spécifique de TNFα comprend au moins une amorce d'amplification spécifique du gène cible TNFα et/ou le réactif spécifique de GATA 3 comprend au moins une amorce d'amplification spécifique du gène cible GATA 3.
Selon un autre mode de réalisation, le réactif spécifique comprend au moins une sonde d'hybridation spécifique d'un gène cible. Préférentiellement, le réactif spécifique de l'IL10 comprend au moins une sonde d'hybridation spécifique du gène cible IL-10, le réactif spécifique de TGFβ comprend au moins une sonde d'hybridation spécifique du gène cible TGFβ, le réactif spécifique de HMG1 comprend au moins une sonde d'hybridation spécifique du gène cible HMG1, le réactif spécifique de T-bet comprend au moins une sonde d'hybridation spécifique du gène cible T-bet, le réactif spécifique de l'IL-1β comprend au moins une sonde d'hybridation spécifique du gène cible IL-1β , le réactif spécifique de TNFα comprend au moins une sonde d'hybridation spécifique du gène cible TNFα et/ou le réactif spécifique de GATA 3 comprend au moins une sonde d'hybridation spécifique du gène cible GATA 3. Cette sonde peut être une sonde de détection ou une sonde de capture, telle que notamment une sonde de capture immobilisée sur une biopuce.
La détermination de l'expression des gènes cibles par l'analyse de l'expression des ARNm est préférentiellement concernée. Lors de l'étape c), on déterminé, en une seule étape, c'est à dire à partir d'un même prélèvement et par une seule analyse (à l'aide de dosage répétés ou de mesure en une seule étape), l'expression d'au moins deux gènes cibles. Les gènes sont ensuite analysés de manière globale pour un patient dont on ne connaît pas le pronostic de son syndrome septique, et en comparant avec des valeurs d'expression moyenne connues d'un même panel de gènes cibles de patients de bon pronostic et des valeurs d'expression moyenne connues d'un même panel de gènes cibles de patients de mauvais pronostic, on peut donc déterminer si le patient est de bon ou de mauvais pronostic afin de lui proposer un traitemen adapté.
Selon un mode préféré de réalisation, on dispose, lors de l'étape b) du procédé, d'au moins trois, préférentiellement d'au moins quatre et encore plus préférentiellement d'au moins cinq réactifs spécifiques sélectionnés parmi les réactifs spécifiques suivants : réactif spécifique du gène d'IL-10, réactif spécifique du gène TGFβ, réactif spécifique du gène HMG1, réactif spécifique du gène T-bet ; réactif spécifique du gène IL-1β, réactif spécifique du gène TNFα, réactif spécifique du gène GATA-3, et on détermine, lors de l'étape c) du procédé, l'expression d'au moins 3 préférentiellement d'au moins 4 et encore plus préférentiellement d'au moins 5 gènes choisis parmi IL-10, TGFβ, HMG1, T-bet, IL-1β, TNFα, GATA-3.
Selon un mode préféré de réalisation, on dispose lors de l'étape b) du procédé, d'au moins trois réactifs spécifiques sélectionnés parmi les réactifs spécifiques suivants : réactif spécifique du gène TGFβ, réactif spécifique du gène HMG1, réactif spécifique du gène IL-1β et on détermine, lors de l'étape c) du procédé , l'expression d'au moins trois gènes cibles choisis parmi : TGFβ, HMG1, IL-1β.
Selon un autre mode préféré de réalisation, on dispose lors de l'étape b) du procédé, d'au moins quatre réactifs spécifiques sélectionnés parmi les réactifs spécifiques suivants : réactif spécifique du gène TGFβ, réactif spécifique du gène HMG1, réactif spécifique du gène IL-1β, réactif spécifique du gène d'IL-10 et on détermine, lors de l'étape c) du procédé, l'expression d'au moins quatre gènes cibles choisis parmi : TGFβ, HMG1, IL-1β, IL-10.
Selon un autre mode préféré de réalisation, on dispose lors de l'étape b) du procédé, d'au moins cinq réactifs spécifiques sélectionnés parmi les réactifs spécifique suivants : réactif spécifique du gène TGFβ, réactif spécifique du gène HMG1, réactif spécifique du gène IL-1β, réactif spécifique du gène d'IL-10, réactif spécifique du gène T-bet et on détermine, lors de l'étape c) du procédé selon l'invention, l'expression d'au moins cinq gènes cibles choisis parmi : TGFβ, HMG1, IL-1β, IL-10, T-bet.

La figure ci-jointe est donnée à titre d'exemple explicatif et n'a aucun caractère limitatif.
Elle permettra de mieux comprendre l'invention.

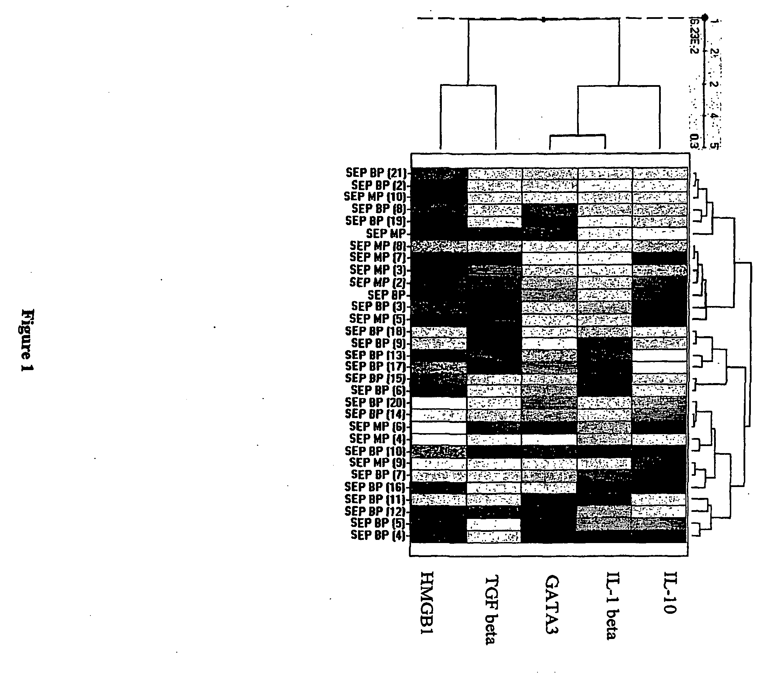

La figure 1 présente un dendogramme obtenu à partir de 31 échantillons issus de patients en syndrome septique de bon pronostic (SEP-BP) ou de mauvais pronostic (SEP-MP), et l'utilisation d'un panel de sondes permettant l'analyse de l'expression de 5 gènes cibles selon l'invention (IL10, IL-1β, GATA-3, TGF-β; HMG1).

Les exemples suivants sont donnés à titre illustratif et n'ont aucun caractère limitatif. Ils permettront de mieux comprendre l'invention.

### Exemple 1 - Etude de l'expression des gènes IL-10, TGFβ, HMG1, T-bet, IL-1β, TNFα, GATA-3 pour le diagnostic d'un syndrome septique

L'étude a été réalisée sur 42 patients ayant développé un syndrome septique, et admis dans le service de réanimation Chirurgicale ou Médicale du centre hospitalier Lyon-Sud. Quinze volontaires sains ont également été utilisés, afin de comparer l'expression des gènes IL-10, TGFβ, HMG1, T-bet, IL-1β, TNFα, GATA-3 dans le sang périphérique de patients sains et de patients en syndrome septique. On distinguait alors un groupe de 15 patients sains (S), et un groupe de 42 patients développant un syndrome septique (SEP)
***Extraction des ARN et synthèse des cDNA** -* Pour chaque patient, l'échantillon biologique était un prélèvement sanguin, été régulièrement obtenu pendant les 11 premiers jours suivant le début du syndrome septique développé par les patients SEP. Un prélèvement a également été effectué selon un même protocole chez les patients sains (S). Ces prélèvements ont été collecté directement dans des tubes PAXGene^{™} Blood RNA (PrcAnalytix, Frankin Lakes, USA).
Après l'étape de prélèvement sanguin et afin d'obtenir une lyse totale des cellules, les tubes ont été laissés à température ambiante pendant 4 h puis conservés à -20°C jusqu'à l'extraction du matériel biologique. Plus préciment, dans ce protocole, les ARN totaux ont été extraits à l'aide des kits PAXGene Blood RNA® (PreAnalytix) en respectant les recommandations du fabriquant. Brièvement, les tubes ont été centrifugés (10 min, 3000g) afin d'obtenir un culot d'acides nucléiques. Ce culot a été lavé et repris dans un tampon contenant de la protéinase K nécessaire à la digestion des protéines (10 min à 55°C). Une nouvelle centrifugation (5 min 19000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set (Qiagen Ltd, Crawley, UK).
Pour chaque extraction, la qualité des ARN totaux a été vérifiée par une électrophorèse sur gel d'agarose et un marquage au bromure d'éthidium. Une réaction de transcription reverse (RT) a été réalisée dans un volume final de 20 µl. L'ARN total (1 µg) a été mélangé avec 1 µl de polyT à 50 µM et 1 µl de dNTP mix (ThermoScript™ RT-PCR system, Invitrogen), puis incubé 5 min à 65°C. Après refroidissement dans la glace, la solution a été mélangée avec 4 µl de 5x cDNA synthesis buffer, 1 µl de RNAse out (40 U/µl), 1 µl d'eau traitée au DEPC et 1 µl de Thermoscript RT (15 U/µl), tous ces produits étant issus du ThermoScript™ RT-PCR system (Invitrogen). La transcription reverse a été effectuée pendant 1 h à 50°C puis stoppée par une incubation de 5 min à 85°C. Pour finir, chaque solution de cDNA a été diluée au 1/10 dans de l'eau DEPC.
***Préparation des standards pour la génération des gammes de quantification*** - Le standard HMG1 a été préparé par une amplification PCR (polymerase chain reaction) conduite jusqu'à saturation. Les amplicons obtenus ont été purifiés (PCR purification kit, Qiagen Ltd) et la présence d'un amplicon unique a été vérifié par électrophorèse sur gel d'agarose et marquage au bromure d'éthidium.
Les standards du gène «de ménage » cyclophilin B et des gènes cibles TGFβ, IL-1β, T-bet, GATA3 et IL-10 ont été obtenus chez Search-LC (Heidelberg, Allemagne).
***Analyse de l'expression des ARNm par PCR en temps réel -*** Les ARNm des gènes cibles TGFβ, IL-1β, T-bet, GATA3, IL-10 et TNFα ont été quantifiés par PCR quantitative en temps réel en utilisant le LightCycler^{™} (Roche). Les réactions PCR ont été effectuées à l'aide du Fast-Start^{™} DNA Master SYBR Green I real-time PCR kit (Roche Molecular Biochemicals). Chaque PCR a été effectuée dans un volume final de 20 µl contenant 1 µl de LC-Fast Start Reaction Mix SYBR Green I, 1 µl de LC-Fast Start DNA Master SYBR Green I/Enzyme (incluant la Taq DNA polymerase, le tampon de réaction et un mélange de deoxynucleotides triphosphate), du MgCl₂ (3 mM concentration finale), les amorces sens et anti-sens (0.5 µM concentration finale ; chacunes obtenues auprès de Search-LC - Heidelberg, Allemagne), et 10 µl de solution de cDNA. Après une étape de dénaturation de 10 min à 95°C, l'amplification a été effectuée à l'aide de 40 cycles d'un protocole de "touch-down" PCR (10 s à 95°C, 10 s d'hybridation à 68-58°C, suivi d'une extension de 16 s à 72°C). A la fin de chaque cycle, la fluorescence émise par le SYBR Green a été mesurée.
Pour confirmer la spécificité de l'amplification, les produits PCR ont systématiquement fait l'objet d'une analyse de courbe de fusion (LightCycler^{™} - Roche).. Pour cela, les produits PCR ont été traités par une température croissante de 58 à 98°C avec une augmentation de 0,1°C/s. Pour chaque produit PCR, un seul pic a été obtenu lors de l'analyse de la courbe, caractérisé par une température de fusion spécifique.
La combinaison des amorces pour la quantification de HMG1 utilisée est celle décrite par Sotiriou et al (Brest Cancer Res. 2002, 4:1-8 correspondant aux amorces sens : 5'- GCG GAC AAG GCC CGT TA-3' et anti-sens : 5'-AGA GGA AGA AGG CCG AAG GA-3' (produit PCR: 119 pb).
Les combinaisons d'amorces nécessaires à la quantification du gène de ménage cyclophilin B et des gènes cibles TGFb, IL- 1b, T-bet, GATA3 et IL-10 ont été obtenu chez Search-LC (Heidelberg, Allemagne).Ainsi, pour le gène de ménage Cyclophilin B, le n° d'accesion Genbank était M60857 et on amplifiait la région 105-338. Pour 1'IL-1b, le n° d'accesion Genbank était M15330 et on amplifiait la région 438-642. Pour TNFa, le n° d'accession Genbank était X01394, et on amplifiait la région 373-783. Pour T-bet , le n° d'accession Genbank était AF241243, et on amplifiait la région 461-669. Pour GATA3, le n° d'accession Genbank était X55037, et on amplifiait la région 1023-1294. Pour TGF-b1, le n° d'accession Genbank était X02812, et on amplifiait la région 1540-1818. Pour IL-10, le n° d'accession Genbank était AY029171, et on amplifiait la région 126-413.
La quantité d'ARNm cible relative à la quantité d'ARNm du gène de ménage cyclophilin B a été analysée par la technique de quantification relative avec le LightCycler Relative Quantification Software (Roche Molecular Biochemicals). La "Second Derivative Maximum Method" du logiciel LightCycler™ (Roche) a été utilisée pour déterminer automatiquement le Crossing Point (Cp) pour chaque échantillon. La valeur du Cp a été définie comme le nombre de cycles pour lequel la fluorescence était significativement différente du bruit de fond.
Cinq dilutions en séries au 1/10 ont été réalisées en quach-uplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

### Résultats obtenus

Les résultats obtenus sont présentés dans le tableau 1 ci dessous.

**Tableau 1**

| | **Patients S** | **Patients SEP** | **p** |
|---|---|---|---|
| **IL-10** | U,0020 ± 0,0002 | U,0259 ± 0,0048 | < 0,001 |
| **TNFα** | 0,0176 ± 0,0025 | 0,0817 ± 0,0150 | < 0,001 |
| **IL.1β** | 0,8121 ± 0,0544 | 1,0114 ± 0,2110 | 0,43 |
| **HMG1** | 0,9736 ± 0,0656 | 1,4807 ± 0,0815 | < 0,001 |
| **GATA3** | 0,0329 ± 0,0026 | 0,0077 ± 0,0010 | < 0,001 |
| **T-bet** | 0,0002 ± 0,0001 | 0,0051 ± 0,0010 | < 0,001 |
| **TGFβ** | 0,5835 ± 0,0607 | 0,2759 ± 0,0200 | < 0,001 |

Ce tableau 1 présente le niveau d'expression des ARNm IL-10, IL-1β, TNFα, HMG1, GATA3, TGFβ et T-bet chez 15 patients sains (S) et 42 patients développant un syndrome septique (SEP), obtenus à partir de prélèvements de sang total obtenu au cours des trois premiers jours suivant le début du syndrome septique. La comparaison entres le groupe des patients S et SEP a été effectuée à l'aide du test U non paramétrique de Mann Whitney et la valeur de probabilité (p) a été calculée. Les résultats sont exprimés par le rapport de quantification relative entre les ARNm du gène cible et les ARNm du gène de ménage cyclophilin B. Les résultats sont exprimés par la moyenne des rapports obtenus pour chacun des groupes de patients. La SEM (standard des écarts à la moyenne) a également été calculée pour chacun des groupes. Les valeurs présentées dans le tableau 1 sont les moyennes obtenues ± la SEM. Les valeurs étaient considérées comme statistiquement différentes lorsque la valeur de p obtenue était inférieure à 0,05.
Les patients SEP présentaient un niveau d'expression des ARNm IL-10 et TNFα significativement augmenté par rapport aux volontaires sains S (respectivement + 10% et + 4%). D'une manière surprenante, les inventeurs ont également montré une surexpression des ARNm d'HMG1 chez les patients SEP (+ 50 %). De plus, les inventeurs ont également mis en évidence que le niveau d'expression des ARNm T-bct était significativement augmenté par rapport aux volontaires sains S (+ 24%). A l'inverse, l'expression des ARNm GATA3 était significativement diminuée chez les patients SEP par rapport aux volontaires sains S (- 70%).
***Conclusion -*** L'étude de l'expression des ARNm des gènes codant T-bet, et GATA3, mais également HMG1 a partir de prélèvements de sang total permet ainsi le diagnostic d'un patient développant un syndrome septique.

### Exemple 2 - Analyse par RT PCR de l'expression des gènes IL-10, TGFβ, HMG1, T-bet, IL-1β, TNFα, GATA-3 pour le pronostic d'un syndrome septique

Dans un deuxième temps, les inventeurs ont recherché un outil pour le pronostic d'un syndrome septique au cours des premières 72 h suivant le début d'un choc septique.
Pour cela, une étude a été réalisée sur 42 patients SEP développant un syndrome septique, et admis dans le service de réanimation Chirurgicale ou Médicale du centre hospitalier Lyon-Sud. Plus précisément, ces patients en syndrome septique ont été sélectionnés au cours des premières 72 h suivant le début d'un choc septique selon les critères d'inclusions de l'American College of Chest Physicians / Society of Critical Care Medicine tels que définis précédemment. Chaque patient a été suivi au cours d'une période maximum de 28 jours. On distinguait alors un groupe de 26 patients développant un syndrome septique de bon pronostic (SEP-BP), et un groupe de 16 patients développant un syndrome septique de mauvais pronostic (SEP-MP).
***Extraction des ARN et synthèse des cDNA -*** Cette étape a été réalisée selon le protocole précédemment décrit dans l'exemple 1.
***Préparation des standards pour la vénération des gammes de quantification -*** Cette étape a été réalisée selon le protocole précédemment décrit dans l'exemple 1.
***Analyse de l'expression des ARNm par PCR en temps réel -*** Cette étape a été réalisée selon le protocole précédemment décrit dans l'exemple 1.

### Résultats obtenus

Les résultats obtenus sont présentés dans le tableau 2 ci après.

**Tableau 2**

| | **Patients SEP-MP** | **Patients SEP-BP** | **p** |
|---|---|---|---|
| **IL-10** | 0,042 ± 0,010 | 0,016 ± 0,003 | 0,1 |
| **TNFα** | 0,103 ± 0,028 | 0,069 ± 0,017 | 0,22 |
| **IL-1β** | 1,147 ±0,530 | 0,928 ± 0,117 | 0,14 |
| **HMG1** | 1,809 ± 0,142 | 1,278 ± 0,077 | 0,01 |
| **GATA3** | 0,006 ± 0,001 | 0,009 ± 0,001 | 0,25 |
| **T-bet** | 0,003 ± 0,001 | 0,006 ± 0,001 | 0,18 |
| **TGFβ** | 0,244 ± 0,022 | 0,296 ± 0,033 | 0,28 |

Ce tableau présente le niveau d'expression des ARNm IL-10, IL-1β, TNFα, HMG1, GATA3, TGFβ et T-bet chez des patients de bon pronostic (SEP-BP) et de mauvais pronostic (SEP-MP) obtenus à partir de prélèvements de sang total obtenus au cours des trois premiers jours suivant le début du choc septique. La comparaison entre le groupe des patients de bon pronostic et de mauvais pronostic a été effectuée à l'aide du test *U* non paramétrique de Mann Whitney et la valeur de probabilité (p) a été calculée. Les résultats sont exprimés par le rapport de quantification relative entre les ARNm du gène cible et les ARNm du gène de ménage cyclophilin B. Les résultats sont exprimés par la moyenne des rapports obtenus pour chacun des groupes de patients. La SEM (standard des écarts à la moyenne) a également été calculée pour chacun des groupes. Les valeurs présentées dans le tableau 2 sont les moyennes obtenues ± la SEM. Les valeurs étaient considérées comme statistiquement différentes lorsque la valeur de p obtenue était inférieure à 0,05.
Les patients de mauvais pronostic présentaient un niveau d'expression des ARNm IL-10, TNFα et HMG1 augmenté par rapport aux patients de bon pronostic. La différence était statistiquement significative pour l'IL-10 et HMG1. A l'inverse de l'IL-10, du TNFα et de HMG1, les patients de mauvais pronostic avaient tendance à présenter des niveaux d'expression des ARNm GATA3, T-bet, IL-1β et TGFβ plus faibles que les patients de bon pronostic.
Les inventeurs ont mis en évidence que l'étude d'un profil d'expression de plusieurs gènes permet d'obtenir un outil de pronostic du syndrome septique, et notamment du choc septique.
A ce titre, le tableau ci dessous présente une analyse de clustering hiérarchique de 36 patients SEP-BP et SEP-MP en utilisant l'expression de 2 à 5 gènes mesurés au cours des trois premiers jours suivant le début du choc septique. La fonction de clustering hiérarchique du logiciel Spofire a été utilisée pour faire cette analyse. Les résultats utilisés pour l'analyse sont exprimés par le rapport de quantification relative entre les ARNm du gène cible et les ARNm du gène de ménage cyclophilin B. Afin de tenir compte des différences constitutives d'expression entre les gènes, les niveaux d'expression de chaque gène ont été normalisés en calculant une variable centrée réduite. Pour chacune des analyses effectuées, 2 sous clusters ont été obtenus, l'un regroupant essentiellement des patients de mauvais pronostic (SEP-MP), et l'autre, des patients de bon pronostic (SEP-BP). Le tableau 3 représente, pour chacun des 2 groupes, le pourcentage de patients clusterisés ensemble, c'est à dire le pourcentage de patients classé MP qui étaient effectivement de mauvais pronostic, et le pourcentage de patients classé BP qui étaient effectivement de bon pronostic.

**Tableau 3**

| **Gènes analysés** | **% de patients SEP-MP bien classés** | **% de patients SEP-BP bien classés** |
|---|---|---|
| TGFβ, HMG1 | 82 | 68 |
| TGFβ, IL-1β, HMG1 | 82 | 72 |
| IL-10, TGFβ, IL-1β, HMG1 | 82 | 80 |
| IL-10, TGFβ, IL-1β, HMG1, T-bet | 82 | 80 |

Ces résultats montrent que l'étude simultanée de l'expression de 2 gènes choisis parmi IL-10, TGFβ, IL-1β, HMG1, T-bet TNFα, GATA3, et notamment TGFβ et HMG1, permet de bien classer plus de 80% des patients SEP-MP. Des résultats comparables étaient obtenus lors de l'étude simultanée de l'expression de 3 gènes choisis parmi IL-10, TGFβ, IL-1β, HMG1, T-bet, TNFα, GATA3, et notamment TGFβ, IL-1β et HMG1, l'étude simultanée de l'expression de 4 gènes choisis parmi IL-10, TGFβ, IL-1β, HMG1, T-bet, TNFα, GATA3, et notamment TGFβ, IL-1β, IL-10, et HMG1, l'étude simultanée de l'expression de 5 gènes choisis parmi Il-10, TGFβ, IL-1β, HMG1, T-bet, TNFα, GATA3, et notamment TGFβ, IL-1β, IL-10, T-bet et HMG1.
***Conclusion** -* L'analyse du profil d'expression d'au moins deux gènes choisis parmi IL-10, TGFβ, IL-1β, HMG1, T-bet, TNFα, GATA3 à partir de prélèvements de sang total permet ainsi un pronostic précoce de la survie des patients présentant un syndrome septique, et développant notamment un choc septique. Plus particulièrement, l'analyse de l'expression d'un panel de gènes comprenant au moins IL-10, IL1-β, TGF-β, HMG-1 permet de discriminer très efficacement les patients de bons pronostics, des patients de mauvais pronostics. Ceci peut permettre au médecin réanimateur d'identifier rapidement les patients de mauvais pronostic et donc d'orienter la prise en charge du patients vers l'utilisation de traitements lourds et coûteux.

### Exemple 3 - Analyse sur puce à ADN de l'expression des gènes IL-10, TGFβ, EMG1, T-bet, IL-1β, TNFα, GATA-3 pour le pronostic d'un syndrome septique

Les résultats présentés dans l'exemple 2 ont été validés par l'utilisation d'une autre technique, appliquée sur de nouveaux échantillons de patients. Pour cela , une étude a été réalisée sur 31 patients développant un syndrome septique, et admis dans le service de réanimation Chirurgicale ou Médicale du centre hospitalier Lyon-Sud. Plus précisément, ces patients en syndrome septique ont été sélectionnés entre le deuxième et le quatrième jours suivant le début d'un choc septique selon les critères d'inclusions de l'American College of Chest Physicians / Society of Critical Care Medicine tels que définis précédemment. Chaque patient a été suivi au cours d'une période maximum de 28 jours. On distinguait alors un groupe de 21 patients développant un syndrome septique de bon pronostic (SEP-BP), et un groupe de 10 patients développant un syndrome septique de mauvais pronostic (SEP-MP).
***Extraction des ARN -*** Cette étape a été réalisée selon le protocole précédemment décrit dans l'exemple 1.
***Analyse de l'expression des ARNm sur puce à ADN-*** une synthèse d'ADNc puis d'ADN double brin a été effectuée à partir des ARN extraits. Les ADN double brin ont été ensuite utilisés comme base pour une transcription in vitro au cours de laquelle le marquage des cRNA est effectué. Les ARNc ont ensuite été hybridés sur la puce. Le scanner permettait la lecture de la fluorescence.

### Résultats obtenus

Les résultats obtenus sont présentés dans la figure 1. On retrouve sur le dendograme 31 colonnes correspondant aux 31 échantillons de patients, et 5 lignes correspondant aux 5 sondes utilisées pour l'analyse de l'expression des 5 gènes cibles. Les échantillons, ainsi que les gènes ayant un profil d'expression comparable, mis en évidence par une corrélation de type Pearson, ont été placés côte à côte. Les échantillons ont été classés selon la méthode de moyenne non-pondérée (Spotfire Decision Site for Functional Genomics V7.1, manual) alors que les gènes ont été classés selon la valeur moyenne d'expression obtenue dans l'ensemble des échantillons. Après normalisation des signaux de fluorescence générés par le logiciel Microarray Suite (MAS5.0, Affymetrix), le niveau d'expression est représenté par différents niveaux de couleur. Ainsi, la couleur blanche correspond à un faible niveau d'expression, la couleur grise correspond à un niveau d'expression intermédiaire, alors que la couleur noire correspond à un fort niveau d'expression. La longueur des branches du dendograme est corrélée au profil d'expression et la ligne en pointillée qui divise le dendograme permet de distinguer deux groupes de patients : un premier groupe de patients de mauvais pronostic "SEP-MP" et un deuxième groupe de patients de bon pronostic "SEP-BP".
Ces résultats montraient que l'étude simultanée de l'expression de 5 gènes choisis parmi IL-10, TGFβ, IL-1β, HMG1, T-bet, TNFα, GATA3, et notamment TGFβ, IL-1β, IL-10, GATA-3 et HMG1 permettent de bien classer 70% des patients.
Les inventeurs ont ainsi mis en évidence que l'étude d'un profil d'expression de plusieurs gènes tel que défini dans l'exemple 2 pour des patients au cours des premières 72 h suivant le début d'un choc septique, était également applicable pour des patients entre le deuxième et quatrième jours suivant le debut d'un choc septique.

***Conclusion -*** L'analyse du profil d'expression d'au moins deux gènes choisis parmi IL-10, TGFβ, IL-1β, HMG1, T-bet, TNFα, GATA3 à partir de prélèvements de sang total permet ainsi un pronostic de la survie des patients au cours des premières 72 h suivant le début d'un choc septique, mais aussi entre le deuxième et quatrième jours suivant le debut d'un choc septique Plus particulièrement, l'analyse de l'expression d'un panel de gènes comprenant au moins IL-10, IL1-β, TGF-β, HMG-1 permet de discriminer très efficacement les patients de bons pronostics, des patients de mauvais pronostics, en tout debut de choc septique, comme quelques jours après. Ceci peut permettre au médecin réanimateur d'identifier rapidement les patients de mauvais pronostic et donc d'orienter la prise en charge du patients vers l'utilisation de traitements lourds et coûteux.

## Revendications

1. Procédé de diagnostic et/ou de pronostic d'un syndrome septique selon lequel:
a. on dispose d'un échantillon biologique du patient et on extrait les acides nucléiques de l'échantillon biologique
b. on dispose d'au moins cinq réactifs spécifiques sélectionnés parmi les réactifs spécifiques suivants: réactif spécifique du gène cible IL-10, réactif spécifique du gène cible TGFβ, réactif spécifique du gène cible HMG1 réactif spécifique du gène cible IL-1β, réactif spécifique du gène cible T-bet
c. on détermine l'expression d'au moins cinq gènes cibles choisis parmi : IL-10, TGFβ, HMG1, T-bet, IL-1β

2. Procédé selon la revendication 1 dans lequel l'échantillon biologique est un échantillon sanguin.

3. Procédé selon l'une quelconque des revendications 1 à 2 dans lequel le réactif spécifique de l'IL10 comprend au moins une amorce d'amplification spécifique du gène cible IL-10, le réactif spécifique de TGFβ comprend au moins une amorce d'amplification spécifique du gène cible TGFβ, le réactif spécifique de HMG1 comprend au moins une amorce d'amplification spécifique du gène cible HMG1, le réactif spécifique de l'IL-1β comprend au moins une amorce d'amplification spécifique du gène cible IL-1β et/ou le réactif spécifique de T-bet comprend au moins une amorce d'amplification spécifique du gène cible T-bet

4. Procédé selon l'une quelconque des revendications 1 à 2 dans lequel le réactif spécifique de l'IL10 comprend au moins une sonde d'hybridation spécifique du gène cible IL-10, le réactif spécifique de TGFβ comprend au moins une sonde d'hybridation spécifique du gène cible TGFβ, le réactif spécifique de HMG1 comprend au moins une sonde d'hybridation spécifique du gène cible HMG1, le réactif spécifique de l'IL-1β comprend au moins une sonde d'hybridation spécifique du gène cible IL-1β et/ou le réactif spécifique de T-bet comprend au moins une sonde d'hybridation spécifique du gène cible T-bet

## Claims

1. A method for diagnosing and/or predicting a septic syndrome, wherein:
a. a biological sample from the patient is made available and biological material is extracted from the biological sample
b. at least five specific reagents which are selected from the following specific reagents are made available: reagent which is specific for the target gene IL-10, reagent which is specific for the target gene TGFβ, reagent which is specific for the target gene HMG1, reagent which is specific for the target gene IL-1β, reagent which is specific for the target gene T-bet
c. the expression of at least four target genes selected from: IL-10, TGFβ, HMG1, T-bet, IL-1β is determined.

2. The method as claimed in claim 1, wherein the biological sample is a blood sample.

3. The method as claimed in any one of claims 1 to 2, wherein the reagent which is specific for IL-10 comprises at least one amplification primer which is specific for the target gene IL-10, the reagent which is specific for TGFβ comprises at least one amplification primer which is specific for the target gene TGFβ, the reagent which is specific for HMG 1 comprises at least one amplification primer which is specific for the target gene HMG1, the reagent which is specific for IL-1β comprises at least one amplification primer which is specific for the target gene IL-1β, and/or the reagent which is specific for T-bet comprises at least one amplification primer which is specific for the target gene T-bet.

4. The method as claimed in any one of claims 1 to 2, wherein the reagent which is specific for IL10 comprises at least one hybridization probe which is specific for the target gene IL-10, the reagent which is specific for TGFβ comprises at least one hybridization probe which is specific for the target gene TGFβ, the reagent which is specific for HMG1 comprises at least one hybridization probe which is specific for the target gene HMG1, the reagent which is specific for IL-1β comprises at least one hybridization probe which is specific for the target gene IL-1β and/or the reagent which is specific for T-bet comprises at least one hybridization probe which is specific for the target gene T-bet.

## Patentansprüche

1. Verfahren zur Diagnose und/oder Prognose eines septischen Syndroms, bei dem man:
a. über eine biologische Probe des Patienten verfügt und die Nukleinsäuren aus der biologischen Probe extrahiert,
b. über mindestens fünf spezifische Reagenzien verfügt, die aus folgenden spezifischen Reagenzien ausgewählt sind: für das IL-10-Zielgen spezifisches Reagenz, für das TGFβ-Zielgen spezifisches Reagenz, für das HMG1-Zielgen spezifisches Reagenz, für das IL-1β-Zielgen spezifisches Reagenz, für das T-bet-Zielgen spezifisches Reagenz,
c. die Expression von mindestens fünf Zielgenen bestimmt, die ausgewählt sind aus: IL-10, TGFβ, HMG1, T-bet, IL-1β.

2. Verfahren nach Anspruch 1, bei dem die biologische Probe eine Blutprobe ist.

3. Verfahren nach einem der Ansprüche 1 bis 2, bei dem das für IL-10 spezifische Reagenz mindestens einen Primer zur spezifischen Amplifikation des IL-10-Zielgens umfasst, das für TGFβ spezifische Reagenz mindestens einen Primer zur spezifischen Amplifikation des TGFβ-Zielgens umfasst, das für HMG1 spezifische Reagenz mindestens einen Primer zur spezifischen Amplifikation des HMG1-Zielgens umfasst, das für IL-1β spezifische Reagenz mindestens einen Primer zur spezifischen Amplifikation des IL-1β-Zielgens umfasst und/oder das für T bet spezifische Reagenz mindestens einen Primer zur spezifischen Amplifikation des T-bet-Zielgens umfasst.

4. Verfahren nach einem der Ansprüche 1 bis 2, bei dem das für IL-10 spezifische Reagenz mindestens eine für das IL-10-Zielgen spezifische Hybridisierungssonde umfasst, das für TGFβ spezifische Reagenz mindestens eine für das TGFβ-Zielgen spezifische Hybridisierungssonde umfasst, das für HMG1 spezifische Reagenz mindestens eine für das HMG1-Zielgen spezifische Hybridisierungssonde umfasst, das für IL-1β spezifische Reagenz mindestens eine für das IL-1β-Zielgen spezifische Hybridisierungssonde umfasst und/oder das für T bet spezifische Reagenz mindestens eine für das T-bet-Zielgen spezifische Hybridisierungssonde umfasst.
